# EUROPÄISCHE PATENTSCHRIFT

(11) **EP 2 397 069 B1**
(45) Veröffentlichungstag und Bekanntmachung des Hinweises auf die Patenterteilung: **27.05.2020**
(21) Anmeldenummer: 11168234.0
(22) Anmeldetag: 31.05.2011
(51) Int. Cl.: A61B 3/16, A61B 3/107

(54) **Analyse des intraokularen Drucks**
Analysis of intraocular pressure
Analyse de la pression intra-oculaire

(30) Priorität: 28.10.2010 DE 102010049633; 28.10.2010 DE 102010049634; 21.06.2010 EP 10166681
(43) Veröffentlichungstag der Anmeldung: 21.12.2011
(73) Patentinhaber: Oculus Optikgeräte GmbH, 35582 Wetzlar-Dutenhofen (DE)
(72) Erfinder: Köst, Gert, 30159 Hannover (DE); Steinmüller, Andreas, 35435 Wettenberg (DE)
(74) Vertreter: advotec.

(56) Entgegenhaltungen:
- EP-A1- 1 692 998
- US-A- 3 406 681
- US-A1- 2005 030 473
- US-A1- 2007 121 120

## Beschreibung

Die Erfindung betrifft ein ophthalmologisches Analyseverfahren zur Messung eines intraokularen Drucks in einem Auge mit einem Analysesystem, sowie ein derartiges Analysesystem, gebildet aus einer Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei mit dem Beobachtungssystem Schnittbilder der unverformten und/oder verformten Hornhaut entlang einer Sehachse des Auges aufgenommen werden, und einer Analyseeinrichtung, mit der aus den Schnittbildern der Hornhaut der intraokulare Druck abgeleitet wird, wobei in der Analyseeinrichtung aus den Schnittbildern der Hornhaut eine Struktureigenschaft der Hornhaut abgeleitet wird.

Derartige Analyseverfahren und -systeme sind hinreichend bekannt und dienen primär einer möglichst genauen, berührungslosen Messung eines intraokularen Drucks in einem Auge. Dazu wird beispielsweise ein Non-Kontakt-Tonometer verwandt, mit Hilfe dessen ein Luftstoß auf das zu untersuchende Auge appliziert wird, wobei eine Stärke des Luftstoßes so gewählt wird, dass die Hornhaut des Auges unter Ausbildung einer konkaven Oberflächenform eingedrückt wird. Vor Erreichen eines Maximums einer Verformung der Hornhaut bzw. vor einem Einklappen der Hornhaut in Richtung der Augenlinse, bildet die Hornhaut kurzzeitig eine plane Fläche aus, die als sogenannter erster Applanationspunkt bezeichnet wird. Nach der maximalen Auslenkung der Hornhaut und einem Zurückklappen derselben in den ursprünglichen Zustand, wird ein ebensolcher zweiter Applanationspunkt von der Hornhaut durchlaufen. Durch eine Inbezugsetzung eines Drucks des Luftstoßes mit einem zeitlichen Verlauf der Applanation der Hornhaut ist es nun möglich, einen intraokularen Druck zu ermitteln. Die mit dem Non-Kontakt-Tonometer ermittelten Messwerte werden in Relation zu Vergleichsmesswerten gesetzt, welche mit einem relativ genauer messenden Applanationstonometer bzw. Kontakt-Tonometer ermittelt wurden, so dass als ein Ergebnis ein dem tatsächlichen intraokularen Druck angenäherter Augeninnendruck abgeleitet werden kann.

Jedoch ist ein mit einem Non-Kontakt-Tonometer gemessener intraokularer Druck gegenüber einer Druckmessung mit einem Applanationstonometer noch nicht hinreichend genau, da eine Messung unter anderem von der Hornhaut verfälscht wird. Um eine Messgenauigkeit zu verbessern, wurde daher versucht, den Einfluss der Hornhaut auf die Messung zu berücksichtigen, beispielsweise durch eine Dickenmessung oder eine Messung von Hornhautradien vor Beginn der Non-Kontakt-Tonometermessung. Auch ist es bekannt, einen Elastizitätsmodul bzw. Youngschen-Modul als eine biomechanische Materialeigenschaft der Hornhaut zu berücksichtigen und die betreffende Messung mit einem entsprechenden Berechnungsfaktor zu korrigieren. Dabei wird bei allen Messungen, auch bei unterschiedlichen Augen, davon ausgegangen, dass der Elastizitätsmodul immer gleich groß und somit konstant ist. Weiter wird davon ausgegangen, dass eine Hornhaut einen in allen Bereichen der Hornhaut gleich großen Elastizitätsmodul aufweist. Eine derartige Einbeziehung eines Elastizitätsmoduls in einer Non-Kontakt-Tonometermessung hat den Nachteil, dass diese Materialeigenschaft bzw. dieser Materialkennwert zur Charakterisierung einer Zugbelastung dient, wie Sie bei einer Non-Kontakt-Tonometermessung nicht auftritt. Weiter variiert ein Elastizitätsmodul von Auge zu Auge individuell und auch, in Abhängigkeit von den jeweiligen Hornhautbereichen, innerhalb der Hornhaut selbst. Eine Einbeziehung derartiger Materialkennwerte und eine Berechnung des Messergebnisses können daher noch nicht zu befriedigend genauen Messergebnissen führen.

Weiter ist es bekannt, die biomechanischen Eigenschaften einer Hornhaut während einer Non-Kontakt-Tonometermessung in diese mit einzubeziehen bzw. diese so bereits während der Messung zu ermitteln. Dazu wird ein Luftstoß auf eine Hornhaut aufgebracht, wobei ein Pumpendruck während des Verlaufs der Messung mittels eines Druckaufnehmers kontinuierlich erfasst wird. Weiter wird ein zeitlicher Verlauf der Messung erfasst sowie ein erster und ein zweiter Applanationspunkt der Hornhaut optisch detektiert. Ein intraokularer Druck kann nun beispielsweise durch eine Bestimmung der zum Zeitpunkt der ersten und zweiten Applanation herrschenden Drücke abgeleitet werden, insbesondere da die zur Biegung der Hornhaut notwendigen Kräfte beim Ein- bzw. Ausklappen der Hornhaut als gleich groß angenommen werden und sich somit gegenseitig aufheben. Folglich resultiert ein intraokularer Druck aus einem Mittelwert der zum Einklappen und Ausklappen der Hornhaut aufgewendeten Kraft, aufgebracht durch den Luftstoß.

Alternativ ist es bekannt, eine Hysterese zwischen dem erstem und dem zweitem Applanationspunkt zu bestimmen und auf Basis der Hysteresemessung den intraokularen Druck abzuleiten bzw. zu korrigieren. Bei der Hysteresemessung wird der erste und zweite Applanationspunkt optisch detektiert und zum Zeitverlauf einer Druckkurve einer Pumpe in Relation gesetzt, das heißt für jeden Applanationspunkt wird ein zugehöriger Zeitwert sowie ein Druckwert ermittelt. Da ein Einklappen der Hornhaut bzw. ein Erreichen des ersten Applanationspunktes bei einem höheren Druckwert erfolgt, als ein Ausklappen bzw. ein Erreichen des zweiten Applanationspunktes, kann dieser Druckunterschied zur Bestimmung der Hysterese als eine Materialeigenschaft der Hornhaut herangezogen werden.

Nachteilig bei diesen Messverfahren ist, dass eine durch einen Luftstoß bewirkte Bewegung der Hornhaut dynamischen Effekten unterliegt, welche derartige Zeit-/Druckmessungen verfälschen können, insbesondere da die dynamischen Effekte bei den beschriebenen Non-Kontakt-Tonometermessungen nicht berücksichtigt werden können. Um derartige, unerwünschte Schwingungen der Hornhaut zu vermeiden, wird eine Geschwindigkeit des Luftstoßes soweit als Möglich minimiert, um ein Messergebnis nicht durch eine unerwünschte Hornhautbewegung zu verfälschen. Weiter ist es notwendig eine Synchronität zwischen Beginn des Luftstoßes und der erforderlichen Zeitmessung herzustellen. Durch eine mechanische, zur Ausbildung eines Luftstoßes verwendeten Pumpe, wie beispielsweise eine Kolbenpumpe, kann diese Zeitsynchronität jedoch, beispielsweise infolge von Massenträgheit oder -reibung, nicht genau erreicht und somit ein Messergebnis verfälscht werden. Auch wird, wie zuvor bereits erwähnt, der Luftstoß drucküberwacht, das heißt nach Bedarf im Verlauf der Messung beeinflusst. So wird nach einem Überschreiten des ersten Applanationspunktes der Luftstoß vermindert bzw. abgeschaltet, um ein übermäßiges Eindrücken der Hornhaut zu verhindern. Dazu ist jedoch ebenfalls eine kontinuierliche Drucküberwachung eines Pumpendrucks sowie eine Überwachung eines zeitlichen Verlaufs desselben relativ zu den Zeitpunkten der ersten und zweiten Applanation notwendig, was eine Einbeziehung einer Reihe von möglichen, einer Messung verfälschenden Fehlerquellen, zur Folge hat. Insgesamt sind daher die aus dem Stand der Technik bekannten Analyseverfahren und -systeme mit voneinander abhängiger, paralleler Druck- und Zeitmessung bei gleichzeitiger Detektion der Applanationspunkte gegenüber einer Messung mit einem Kontakt-Tonometer noch vergleichsweise ungenau.

Aus der EP 1 692 998 A1 ist ein Non-Kontakt-Tonometer mit einem Scheimpflugsystem zur Gewinnung von Schnittbildern der Hornhaut und mit einer Analyseeinrichtung, mittels der aus Bildinformationen bzw. den Schnittbildern der Hornhaut der intraokulare Druck abgeleitet werden kann, bekannt.

Aus der US 3,406,681 ist ein Verfahren zur Spannungsmessung einer Hornhaut eines Auges bekannt. Die Spannungsmessung steht dabei insbesondere in einem Zusammenhang mit einer Messung eines intraokularen Drucks des Auges. Die Messung des intraokularen Drucks erfolgt durch eine Beaufschlagung der Hornhaut mit einem von einer Pumpe erzeugten Druck und einer Messung des Drucks mit einem Manometer. Weiter wird eine spannungsoptische Darstellung der Hornhaut durch monochromatisches, polarisiertes Licht erzeugt. Das Auge wird dazu von einer Vorderseite her mit dem polarisierten Licht beleuchtet, wobei die sich ergebenden spannungsoptischen Muster ebenfalls von der Vorderseite her beobachtet werden.

Der vorliegenden Erfindung liegt daher die Aufgabe zugrunde, ein ophthalmologisches Analyseverfahren zur Messung eines intraokularen Drucks in einem Auge sowie ein derartiges Analysesystem vorzuschlagen, mit dem durch Erfassung eines alternativen Strukturaspekts der Hornhaut eine vergleichsweise verbesserte Messgenauigkeit erzielbar ist.

Diese Aufgabe wird durch ein ophthalmologisches Analyseverfahren mit den Merkmalen des Anspruchs 1 und ein Analysesystem mit den Merkmalen des Anspruchs 14 gelöst.

Bei dem erfindungsgemäßen, ophthalmologischen Analyseverfahren zur Messung eines intraokularen Drucks in einem Auge mit einem Analysesystem, umfasst das Analysensystem eine Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, ein Beobachtungssystem, mit dem die Verformung der Hornhaut beobachtet und aufgenommen wird, wobei mit dem Beobachtungssystem Schnittbilder der unverformten und/oder verformten Hornhaut entlang einer Sehachse des Auges aufgenommen werden, und eine Analyseeinrichtung, mit der aus den Schnittbildern der Hornhaut der intraokulare Druck abgeleitet wird, wobei in der Analyseeinrichtung aus den Schnittbildern der Hornhaut eine Struktureigenschaft der Hornhaut abgeleitet wird, wobei als eine Struktureigenschaft eine Spannung der Hornhaut abgeleitet wird, wobei Spannungen im Material der Hornhaut visualisiert werden, wobei als ein Schnittbild jeweils eine spannungsoptische Darstellung der Hornhaut verwendet wird, wobei aus Spannungslinien der spannungsoptischen Darstellung die Struktureigenschaft der Hornhaut abgeleitet wird, wobei der intraokulare Druck unter Berücksichtigung der Spannungen der Hornhaut korrigiert wird.

Eine Materialeigenschaft ist im vorliegenden Zusammenhang als eine Eigenschaft definiert, die dem Material unabhängig von äußeren Einflüssen innewohnt. Eine Struktureigenschaft ist eine Eigenschaft, die durch äußere Einflüsse im Material oder auch durch eine Gestalt des Materials beeinflusst ist. Nach der Erfindung ist es vorgesehen, Spannungen der Hornhaut durch die Aufnahme der Schnittbilder zu visualisieren. Dabei kann zwischen Spannungen unterschieden werden, die unabhängig von einem intraokularen Druck, abhängig von einem intraokularen Druck und bedingt durch die Verformung der Hornhaut im Material der Hornhaut vorliegen. Diese Unterscheidung wird dadurch möglich, dass durch die Aufnahme der Schnittbilder Spannungen der unverformten Hornhaut und nachfolgend Spannungen der verformten Hornhaut aufgenommen und visualisiert werden. Je nach Art, Stärke, Richtung oder Verteilung der Spannungen in den Schnittbildern der Hornhaut, wird es möglich den intraokularen Druck unter Berücksichtigung der Spannung zu korrigieren.

Erfindungsgemäß wird der intraokulare Druck unter Berücksichtigung der Struktureigenschaft der Hornhaut abgeleitet. Eine Korrektur des intraokularen Drucks kann insbesondere durch einen Vergleich eines Verhältnisses der Spannungen der unverformten und der verformten Hornhaut an einem definierten Punkt bzw. einer Position der verformten Hornhaut möglich werden. In einem weiteren Schritt des Verfahrens kann vorgesehen sein, die visualisierten Spannungen mit in einer Datenbank, gespeicherten, visualisierten Spannungen zu vergleichen, um den intraokularen Druck zu korrigieren. So ist dann für die in der Datenbank gespeicherten Werte ein objektiver Augeninnendruck oder auch ein entsprechender Korrekturwert bekannt, so dass der objektive intraokulare Druck des gemessenen Auges unter Berücksichtigung der Spannungen der Hornhaut abgeleitet werden kann.

Erfindungsgemäß wird als ein Schnittbild jeweils eine spannungsoptische Darstellung der Hornhaut verwendet. Eine spannungsoptische Darstellung ermöglicht eine einfache Visualisierung einer Spannungsverteilung in lichtdurchlässigen Körpern, wobei eine Verteilung und Größe der mechanischen Spannung an allen Stellen der Hornhaut, oder auch in anderen lichtdurchlässigen Bereichen des Auges, einfach dargestellt und mittels Bildverarbeitung ausgewertet werden kann. Insbesondere können dabei in der Ebene des Schnittbildes auftretende Spannungen visualisiert werden. Quer zur Ebene des Schnittbildes verlaufende Spannungen werden dann nicht berücksichtigt, wobei dies zur Korrektur des intraokularen Drucks auch nicht zwingend notwendig ist.

Erfindungsgemäß wird aus Spannungslinien der spannungsoptischen Darstellung die Struktueigenschaft der Hornhaut abgeleitet. Die Spannungslinien sind besonders gut visuell erkennbar und auch wird es dann möglich zwischen der Struktur- und einer Materialeigenschaft der Hornhaut zu unterscheiden. Bei den Spannungslinien kann zwischen Isochromaten und Isoklinen unterschieden werden, wobei die Isochromaten Spannungslinien mit konstanter Hauptspannungsdifferenz sind und die Isoklinen Spannungstrajektorien der Hornhaut unter einer gegebenen Belastung repräsentieren. So können aus einer Vielzahl von während einer Verformung der Hornhaut gewonnen Schnittbildern, sich aufgrund der durch den Luftstoß bedingten Belastung der Hornhaut verändernde Spannungslinien und aufgrund einer Gestalt der Hornhaut selbst in dieser vorliegende Spannungslinien, welche sich relativ zur Hornhaut nicht wesentlich verändern, unterschieden werden.

Das Analysesystem kann in Art eines Polariskops ausgebildet sein, wobei das Beobachtungssystem dann eine Beleuchtungseinrichtung und eine Kameraeinrichtung umfassen kann, die jeweils einen Polarisator aufweisen, wobei mittels der Beleuchtungseinrichtung das Auge mit linear, zirkular oder elliptisch polarisiertem Licht beleuchtet werden kann. Beispielsweise kann es dann ausreichend sein an der Beleuchtungseinrichtung einen entsprechenden Polarisiationsfilter und an der Kameraeinrichtung einen Polarisationsfilter vorzusehen, um Spannungen im Material der Hornhaut visualisieren zu können. Mit den verschiedenen Arten des polarisierten Lichts können dann verschiedene Effekte für eine geeignete Visualisierung erzielt werden. Beispielsweise sind bei einer Verwendung von zirkular polarisiertem Licht keine Isoklinen sichtbar.

Diesbezüglich ist es auch möglich das Auge mit monochromatischem oder polychromatischem Licht zu beleuchten. Durch eine Verwendung von monochromatischem Licht entstehen dunkle und helle Streifen im Schnittbild der Hornhaut, deren Anordnung Rückschlüsse auf die mechanischen Spannungen der Hornhaut ermöglichen. Polychromatisches Licht ermöglicht weiter eine Farbdarstellung der Streifen bzw. von Spannungslinien.

Alternativ kann zur Anpassung der Visualisierung auch eine Polarisationsrichtung relativ zum Schnittbild gedreht werden.

Als eine weitere Materialeigenschaft kann eine Steifigkeit der Hornhaut abgeleitet werden, wobei der intraokulare Druck dann unter Berücksichtigung der Materialeigenschaften der Hornhaut abgeleitet werden kann. Der Begriff der Steifigkeit ist hier explizit nicht als ein Elastizitätsmodul bzw. Youngscher-Modul zu verstehen, sondern als eine Materialeigenschaft, die durch eine auf das Auge wirkenden Druckbelastung charakterisiert ist bzw. dieser entgegensteht, also den bei einer Tonometermessung tatsächlich auftretenden Lastfall betrifft. Die Steifigkeit ist somit eine richtungsabhängige Kenngröße des Materials der Hornhaut. Weiter ist die Steifigkeit durch das Material der Hornhaut selbst und nicht durch sonstige, äußere Einflüsse bestimmt. Auch können im Material der Hornhaut intrinsische Spannungen wirken die die Steifigkeit der Hornhaut beeinflussen.

Der intraokulare Druck und die Steifigkeit der Hornhaut können als eine unabhängige, die Hornhaut beschreibende, weitere Materialeigenschaft getrennt voneinander bestimmt werden. So kann während einer einzigen Messung durch Aufbringen eines Luftstoßes nach einem herkömmlichen, tonometrischen Verfahren ein erster intraokularer Druck bestimmt werden. Parallel dazu kann aus der Verformung der Hornhaut, die während der Verformung durch das Beobachtungssystem aufgenommen wird, die Steifigkeit der Hornhaut abgeleitet werden. Da die Steifigkeit der Hornhaut einen wesentlichen Einfluss auf ein Verformungsverhalten der Hornhaut bzw. die Messung des ersten intraokularen Drucks des Auges hat, kann der Einfluss der Hornhaut auf die Messung des ersten intraokularen Drucks berücksichtigt werden. So kann der zuvor gemessene, erste intraokulare Druck um den Einfluss der Hornhaut auf die Messung korrigiert werden, so dass ein objektiver intraokularer Druck als ein Ergebnis der Messung abgeleitet werden kann. Die Steifigkeit der Hornhaut entspricht dabei im Wesentlichen näherungsweise einer linearen Funktion von dem ersten, gemessenen subjektiven Intraokularen Druck des Auges sowie einer gemessenen maximalen Amplitude der Verformung der Hornhaut. Bei einem Graph der Funktion der Steifigkeit kann der subjektive intraokulare Druck beispielsweise auf einer Ordinatenachse und die maximale Amplitude der Verformung auf einer Abzissenachse abgebildet sein, wobei die Steifigkeit dann im Wesentlichen eine Form einer Geraden mit negativer Steigung aufweist. Je nach Messwert für die Abzissenachse und Ordinatenachse kann sich bei sich verändernden Messwerten im Wesentlichen eine Parallelverschiebung der Geraden ergeben, woraus jeweils unterschiedliche Steifigkeiten resultieren können. Der objektive intraokulare Druck kann von der ermittelten Steifigkeit abgeleitet werden bzw. kann von der Geraden der Steifigkeit aus einem Schnittpunkt des Wertes für den subjektiven intraokularen Drucks bzw. des Wertes für die maximale Amplitude mit der Geraden für die Steifigkeit abgetragen werden. Bei der Messung kann immer die Steifigkeit der Hornhaut als eine weitere Materialeigenschaft für jede Messung neu ermittelt werden, dass heißt, es wird nicht, wie aus dem Stand der Technik bekannt, von einer für jedes beliebige Auge konstanten Materialeigenschaft ausgegangen.

Weiter kann es besonders vorteilhaft sein, wenn während der Messung bzw. des Verformungsvorgangs der Hornhaut eine Serie bzw. Vielzahl von Schnittbildern der Hornhaut aufgenommen wird. So wird es möglich, eine Verformung der Hornhaut im Detail zu verfolgen und mittels einer Bildverarbeitung der Schnittbilder aus dem Verformungsablauf die entsprechende Materialeigenschaft bzw. einen objektiven intraokularen Druck abzuleiten.

Zur Ableitung einer weiteren Materialeigenschaft kann auch ein Zeitabschnitt zwischen Beginn und Ende der Verformung der Hornhaut gemessen werden. So können insbesondere alle aufgenommenen Schnittbilder jeweils einem bestimmten Zeitpunkt der Messung zugeordnet werden, wodurch ein zeitlicher Ablauf der Verformung nachvollziehbar wird. Insbesondere kann ein Zeitpunkt der ersten und der zweiten Applanation der Hornhaut und somit ein zeitlicher Abstand genau bestimmt werden. So kann auch die Ermittlung dieses Zeitabschnittes zur Bestimmung der betreffenden Materialeigenschaft ausreichend sein. Weiter kann zur Ableitung der Materialeigenschaft ein Zeitabschnitt der gesamten Verformung der Hornhaut herangezogen werden.

Zur Ableitung einer weiteren Materialeigenschaft kann eine Geschwindigkeit der bewegten Hornhaut gemessen werden. Wenn insbesondere der Zeitverlauf einer Verformung der Hornhaut bekannt ist, kann so auch eine Dynamik der Verformung untersucht werden, um besondere dynamische Effekte bei der Verformung in Hinblick auf die betreffende Materialeigenschaft zu bewerten. Beispielsweise kann sich ein Nachschwingen der Hornhaut bei einem Luftstoß somit nicht mehr verfälschend auf ein Messergebnis auswirken, wenn das Nachschwingen bei der Messung berücksichtigt wird. Auch wird so eine Geschwindigkeit eines Luftstoßes in Bezug auf sonst unerwünschte dynamische Effekte für eine Messung beliebig wählbar. Auch ist es möglich von der gemessenen Geschwindigkeit auf eine Eindrücktiefe bzw. maximale Amplitude zu schließen, da zwischen diesen Größen ein funktionaler Zusammenhang besteht.

Ein Entfernungsmaß einer maximalen Verformung der Hornhaut relativ zum Apex der Hornhaut kann aus den Schnittbildern der Hornhaut abgeleitet werden. Folglich kann eine maximale Eindrücktiefe der Hornhaut aus den Schnittbildern der Hornhaut ermittelt werden, wobei ergänzend auch ein Zeitpunkt der maximalen Verformung der Hornhaut zumindest relativ zu einem der Applanationspunkte festgestellt werden kann.

Eine Amplitude eines geometrischen Verformungsverlaufs der Hornhaut kann aus den Schnittbildern der Hornhaut abgeleitet werden. So kann der genaue geometrische Verlauf der Verformung leicht nachvollzogen werden. Das heißt, es kann zu jedem Zeitpunkt der Verformung die zu diesem Zeitpunkt existente, geometrische Gestalt der Verformung aufgenommen werden, so dass der geometrische Verlauf der Verformung in Art eines Films der Verformung erfasst werden kann. Beispielsweise ist auch ein Nachschwingen der Hornhaut nach einem Ausklappen bzw. dem zweiten Applanationspunkt so gut erfassbar.

Auch kann eine Krümmung der unverformten und/oder verformten Hornhaut aus den Schnittbildern der Hornhaut abgeleitet werden. Da die Schnittbilder der Hornhaut auch eine Geometrie derselben beschreiben, insbesondere vor Aufbringen des Luftstoßes, kann die Geometrie der Hornhaut in Verbindung mit der betreffenden Materialeigenschaft der Hornhaut in die Berechnung des objektiven, intraokularen Drucks mit einbezogen werden. Das heißt die Krümmungsradien bzw. eine Kurvatur der Hornhaut kann auf einer äußeren und/oder inneren Hornhautoberfläche aus den Schnittbildern mittels Bildverarbeitung abgeleitet werden. Dabei können die Krümmungsradien bei der unverformten Hornhaut als ein Korrekturfaktor und beispielsweise die Dicke der Hornhaut bei der verformten Hornhaut in die Messung einfließen und als ein Indikator für die Materialeigenschaft herangezogen werden.

Optional kann bei Erreichen eines Applanationspunktes der Hornhaut eine Größe einer ebenen Applanationsfläche gemessen werden. Beispielsweise kann eine Größe der Applanationsfläche bzw. deren Durchmesser und/oder deren Form als ein Indikator für eine Steifigkeit der Hornhaut berücksichtigt werden.

In diesem Zusammenhang kann auch ein Durchmesser d₁ einer ersten Applanationsfläche der Hornhaut und ein Durchmesser dₙ einer von der ersten Applanationsfläche abweichenden Verformungsfläche der Hornhaut abgeleitet werden. Bei der Verformung der Hornhaut mittels des Luftstoßes kann es zu einer vollständigen Applanation der Hornhaut kommen, wobei dann die erste Applanationsfläche mit dem Durchmesser d₁ ausgebildet wird. Die Applanationsfläche kann im Wesentlichen eben sein und im Bereich einer Applanationsebene orthogonal zu einer Sehachse des Auges bzw. einer Geräteachse eines Analysesystems liegen. Während der Verformung der Hornhaut kann in der Hornhaut eine konkave Vertiefung ausgebildet werden, die sich von der ersten Applanationsfläche wesentlich unterscheidet. Ein Vergleich der von der ersten Applanationsfläche abweichenden Verformungsfläche der Vertiefung mit der ersten Applanationsfläche kann eine Definition der Materialeigenschaft der Hornhaut ermöglichen, da die Verformungsfläche in Abhängigkeit der Materialeigenschaft ausgebildet wird. Die weitere Materialeigenschaft betrifft somit keine allgemein bekannte Materialeigenschaft, sondern beruht alleine auf einer Definition zweier voneinander abweichender Geometrien der verformten Hornhaut. Ein Referenzmaßstab für die Abweichung ist dabei die erste Applanationsfläche bzw. der Durchmesser d₁ der ersten Applanationsfläche. Dadurch, dass der Vergleich mit dem Durchmesser dₙ der Verformungsfläche der Hornhaut erfolgt, kann der Vergleich besonders einfach ausgeführt werden. Der Durchmesser dₙ kann insbesondere bei einer Verformungsbewegung der Hornhaut nach Passieren der ersten Applanationsfläche bzw. eines ersten Applanationspunktes besonders einfach bestimmbar sein, da dann die Verformungsfläche eine konkave Gestalt annimmt. Weiter kann vorgesehen sein, dass die Verformungsfläche bzw. der Durchmesser dₙ in einem bestimmten Zeitabschnitt der Verformung relativ zur ersten Applanationsfläche oder auch ein anderer, messbarer Punkt oder eine Position der Hornhaut während der Verformung zur Definition der abweichenden Verformungsfläche der Hornhaut herangezogen wird. Auch können die ermittelte Abweichung und die Relativwerte der betreffenden Durchmesser in einer Datenbank gespeichert und verglichen werden. So kann für die in der Datenbank gespeicherten Werte ein objektiver Augeninnendruck oder auch ein entsprechender Korrekturwert bekannt sein, so dass der objektive intraokulare Druck des gemessenen Auges unter Berücksichtigung der geometrisch definierten Materialeigenschaft der Hornhaut abgeleitet werden kann.

Weiter kann zur Ableitung der weiteren Materialeigenschaft ein Durchmesser d₂ einer Verformungsfläche der Hornhaut bei einer maximalen Verformung der Hornhaut in Richtung einer Sehachse oder Geräteachse bestimmt werden. Die maximale Verformung der Hornhaut kann aus einer Serie von Schnittbildern der verformten Hornhaut bestimmt werden. So wird es möglich für jede Messung einen Zeitpunkt der Verformung bzw. eine Geometrie der Hornhaut zu definieren, der bzw. die als ein Vergleichsmaßstab zur ersten Applanationsfläche der Hornhaut dienen kann. Auch kann der Durchmesser d₂ dann einfach dadurch bestimmt werden, dass dieser als ein Abstand von zwei gegenüberliegenden Punkten in einer Längsschnittebene der Hornhaut im Zustand der maximalen Verformung definiert wird, wobei die Punkte jeweils die dem Analysesystem am nahesten zugewandten Punkte repräsentieren. Diese Punkte können einem Schnittbild entnommen werden und repräsentieren folglich den Durchmesser d₂ der maximalen Verformung bzw. Deformation der Hornhaut.

Zur Ableitung der weiteren Materialeigenschaft kann ein Verhältnis zwischen dem Durchmesser d₁ der ersten Applanationsfläche der Hornhaut und einem Durchmesser d₃ einer zweiten Applanationsfläche der Hornhaut bestimmt werden. Während einer Verformung der Hornhaut mittels des Luftstoßes erfolgt ein Einklappen der Hornhaut unter Ausbildung der ersten Applanationsfläche bis hin zu einer maximalen Verformung der Hornhaut mit einer konkaven Vertiefung sowie nachfolgend ein Ausklappen der Hornhaut unter Ausbildung der zweiten, weitestgehend ebenen Applanationsfläche bis zu einem Erreichen der ursprünglichen Gestalt der Hornhaut. Die zweite Applanationsfläche stellt damit einen in den Schnittbildern gut erkennbaren, geometrischen Referenzpunkt dar, der zur Definition der Materialeigenschaft durch einen Vergleich mit der ersten Applanationsfläche herangezogen werden kann. Insbesondere durch eine mögliche Abweichung der Durchmesser der Applanationsflächen kann eine Materialeigenschaft der Hornhaut definiert bzw. bestimmt werden. Auch können die an die jeweilige Applanationsfläche anschließenden Radien der Hornhaut als ein weiterer Indikator verwendet werden.

Als eine weitere Materialeigenschaft kann ein Schubmodul (G) der Hornhaut abgeleitet werden. Ein Schubmodul kann als eine lineare Materialkenngröße als eine besonders vereinfachte Angabe der Steifigkeit der Hornhaut verwendet werden, insbesondere da ein derartig lineares Materialverhalten unaufwendig im Rahmen der Analyseeinrichtung interpretiert werden kann.

Um neben der Steifigkeit der Hornhaut eine Elastizität als eine weitere Materialeigenschaft der Hornhaut bei der Messung zu berücksichtigen, kann eine Lichtstreuung der Hornhaut aus einem Schnittbild der Hornhaut abgeleitet werden, wobei aus der Lichtstreuung eines einzelnen Schnittbildes als eine weitere Materialeigenschaft die Elastizität der Hornhaut abgeleitet wird. Eine optische Trübung der Hornhaut kann als ein Indikator für eine Materialalterung der Hornhaut verwendet werden, wobei aus einem Alter der Hornhaut Rückschlüsse über deren Elastizität gezogen werden können. So ist eine Hornhaut bei fortschreitender Trübung und somit erhöhter Lichtstreuung vergleichsweise weniger elastisch als eine Hornhaut mit einer geringeren Lichtstreuung. Die Elastizität der Hornhaut kann in diesem Fall als ein individueller Elastizitätsmodul des gemessenen Auges berücksichtigt werden.

Die Messung kann noch weiter dadurch verbessert werden, dass unterschiedlichen Bereichen der Hornhaut jeweils voneinander abweichende Struktur- und Materialeigenschaften zugeordnet werden. So können, bei angenommener, gleicher Dicke der Hornhaut die Materialeigenschaften in unterschiedlichen Bereichen eines Querschnitts der Hornhaut oder in Bezug auf einen Oberflächenbereich der Hornhaut variieren bzw. voneinander verschieden sein.

In einer vorteilhaften Ausführungsform des Analyseverfahrens kann das Beobachtungssystem eine Kamera und eine Beleuchtungseinrichtung in einer Scheimpfluganordnung umfassen, wobei dann mittels der Kamera die Schnittbilder aufgenommen werden können. Das heißt die Kamera kann relativ zu einer optischen Achse einer Spaltbeleuchtungseinrichtung zur Beleuchtung des Auges in einer Scheimpfluganordnung angeordnet sein, so dass ein beleuchtetes Querschnittsbild des Auges mit der Kamera aufgenommen werden kann. Eine Kamera kann beispielsweise auch als eine Hochgeschwindigkeitskamera verwendet werden, die mindestens 4000 Bilder pro Sekunde aufnehmen kann. Auch kann die optische Achse der Spaltbeleuchtungseinrichtung in eine Sehachse des Auges fallen bzw. mit dieser übereinstimmen. Vorzugsweise kann dann auch eine Wirkrichtung des Luftstoßes koaxial zur optischen Achse der Spaltbeleuchtungseinrichtung verlaufen.

Das erfindungsgemäße ophthalmologische Analysesystem zur Messung eines intraokularen Drucks in einem Auge umfasst eine Betätigungseinrichtung, mit der eine Hornhaut des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei mit dem Beobachtungssystem Schnittbilder der unverformten und/oder verformten Hornhaut entlang einer Sehachse des Auges aufgenommen werden können, und eine Analyseeinrichtung, mit der aus den Schnittbildern der Hornhaut der intraokulare Druck abgeleitet werden kann, wobei in der Analyseeinrichtung aus den Schnittbildern der Hornhaut eine Struktureigenschaft der Hornhaut abgeleitet wird, wobei als eine Struktureigenschaft eine Spannung der Hornhaut abgeleitet wird, wobei Spannungen im Material der Hornhaut visualisiert werden, wobei der intraokulare Druck unter Berücksichtigung der Struktureigenschaft der Hornhaut abgeleitet wird, wobei als ein Schnittbild jeweils eine spannungsoptische Darstellung der Hornhaut verwendet wird, wobei aus Spannungslinien der spannungsoptischen Darstellung die Struktureigenschaft der Hornhaut abgeleitet wird, wobei der intraokulare Druck unter Berücksichtigung der Spannungen der Hornhaut korrigiert wird.

Weitere vorteilhafte Ausführungsformen des Analysesystems ergeben sich aus den Merkmalsbeschreibungen der auf den Verfahrensanspruch 1 rückbezogenen Unteransprüche.

Nachfolgend wird eine bevorzugte Ausführungsform der Erfindung unter Bezugnahme auf die beigefügten Zeichnungen näher erläutert.

Es zeigen:
- **Fig. 1a** bis **1e:**: eine Verformung einer Hornhaut eines Auges während einer Messung in einer Längsschnittansicht;
- **Fig. 2:**: eine Diagrammdarstellung von Pumpendruck und -zeit während einer Messung;
- **Fig. 3:**: eine Diagrammdarstellung von gemessenem intraokularen Druck und Verformung der Hornhaut;
- **Fig. 4a bis 4b:**: eine Visualisierung von Spannungen im Material der Hornhaut des Auges.

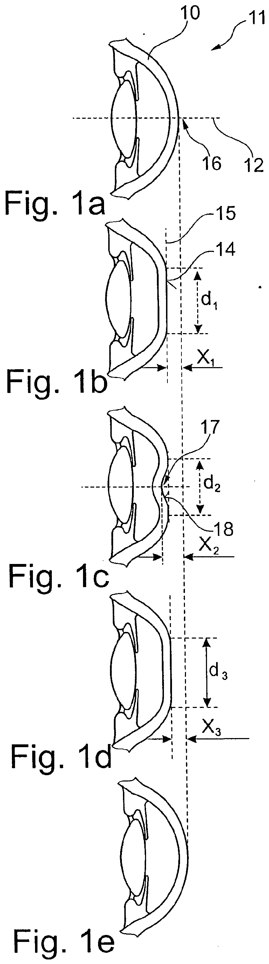

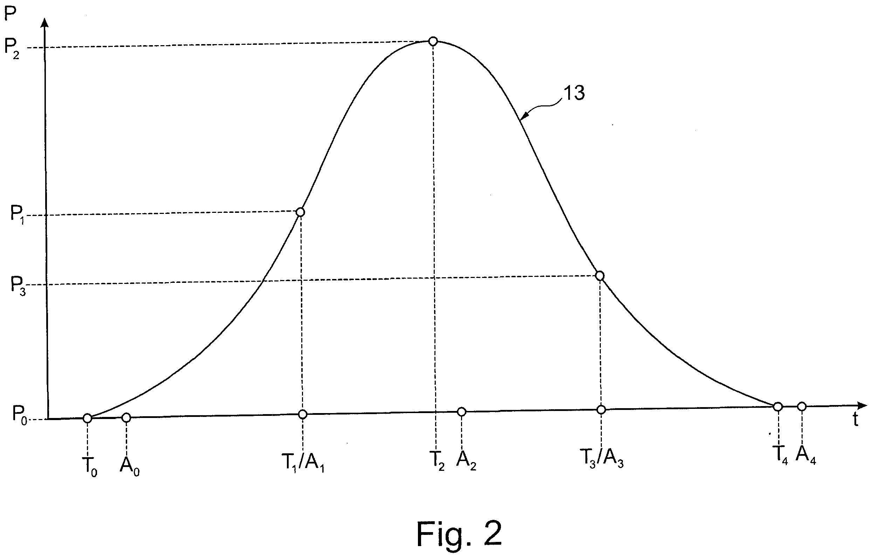

Die **Fig. 1a** bis **1e** zeigen ausgewählte Verformungszustände einer Hornhaut 10 eines Auges 11 während einer einzelnen Messung eines Augeninnendrucks durch ein hier nicht dargestelltes Analysesystem. Die Darstellungen sind jeweils Längsschnittdarstellungen entlang einer Sehachse 12 des Auges 11. **Fig. 2** ist eine Diagrammdarstellung mit einer Zeit t auf der Abzissenachse und einem Pumpendruck p auf der Ordinatenachse. Der Pumpendruck verläuft unabhängig vom Einsatz eines hier nicht dargestellten Beobachtungssystems bzw. einer Scheimpflugkamera mit einer Spaltbeleuchtungseinrichtung in Art einer symmetrischen Glockenkurve 13, beginnend mit einem Druck P₀ bei einem Startpunkt T₀ der Pumpe bis hin zu einem maximalen Pumpendruck P₂ zum Zeitpunkt T₂ und wieder abfallend bis hin zu dem Pumpendruck P₀ in einem Endpunkt T₄. Der durch den Start der Pumpe bei T₀ abgegebene Luftstoß auf die Hornhaut 10 führt zu einer ersten, mittels des Beobachtungssystems aufnehmbaren Verformung der Hornhaut 10 unmittelbar nach dem Zeitpunkt A₀. **Fig. la** repräsentiert die Gestalt der noch nicht verformten Hornhaut 10 zum Zeitpunkt A₀. Mit steigendem Pumpendruck kommt es zum Zeitpunkt A₁ zu einer vollständigen Applanation der Hornhaut 10 gemäß **Fig. 1b****,** wobei wie hier dargestellt, eine Applanationsfläche 14 mit einem Durchmesser d₁ ausgebildet wird, die im Wesentlichen eben ist und in einer Applanationsebene 15 liegt. Die Hornhaut ist dann um ein Maß X₁ vom Apex 16 der Hornhaut 10 entfernt bzw. eingedrückt. Optional und nicht notwendigerweise kann bei diesem sogenannten ersten Applanationspunkt zum Zeitpunkt A₁ ein Pumpendruck P₁ zum übereinstimmenden Zeitpunkt T₁ ermittelt werden. Nach Erreichen des Pumpendrucks P₂ kommt es zu einer maximalen Verformung der Hornhaut 10 zum Zeitpunkt A₂ entsprechend der Darstellung in **Fig. 1c****.** Ein eine maximale Verformung bestimmender Punkt 17 ist dabei um ein Maß X₂ vom Apex 16 der Hornhaut 10 entfernt. Es handelt sich in diesem Fall also um eine maximale Auslenkung einer Amplitude der Verformung. Bei dieser maximalen Amplitude der Verformung wird ein Durchmesser d₂ einer konkaven Verformungsfläche 18 ausgebildet und erfasst. Der Durchmesser d₂ ist durch einen Abstand von zwei gegenüberliegenden Punkten einer Längsschnittebene der Hornhaut 10 definiert, wobei die Punkte jeweils die dem Analysesystem am nächsten zugewandten Punkte der Hornhaut 10 repräsentieren. Nachfolgend kommt es zu einer Rückbewegung bzw. einem Ausschwingen der Hornhaut 10, wobei zum Zeitpunkt A₃ der sogenannte zweite Applanationspunkt gemäß Darstellung in **Fig. 1d** erreicht wird. Hier wird ebenfalls ein Durchmesser d₃ sowie ein Abstand X₃ erfasst. Auch ist es optional möglich einen Pumpendruck P₃ zum übereinstimmenden Zeitpunkt T₃ zu bestimmen. Nach dem Rückgang des Pumpendrucks auf den Ursprungswert P₀ zum Zeitpunkt T₄ gelangt die Hornhaut 10 zum Zeitpunkt A₄ ebenfalls wieder in ihre in **Fig. 1e** dargestellte Ausgangslage. Entsprechend der vorangehenden Beschreibung einer einzelnen Messung eines intraokularen Drucks eines Auges werden die in den **Fig. 1a** bis **1e** dargestellten Verformungszustände der Hornhaut 10, welche durch die jeweiligen mit A₀ bis A₄ gekennzeichneten Zeitpunkte charakterisiert sind, ermittelt. Dabei werden insbesondere unabhängig von einem Pumpendruck p Zeitabstände der betreffenden Zeitpunkte A₀ bis A₄ sowie die Maße bzw. Eindrücktiefen X₁, X₂ und X₃ erfasst, wobei aus diesen Kenngrößen eine Steifigkeit der Hornhaut 10 abgeleitet wird. Ein gemessener intraokularer Druck wird dann durch einen durch die Steifigkeit der Hornhaut bestimmten Wert korrigiert, sodass ein objektiver intraokularer Druck als ein Ergebnis der Messung ausgegeben wird.

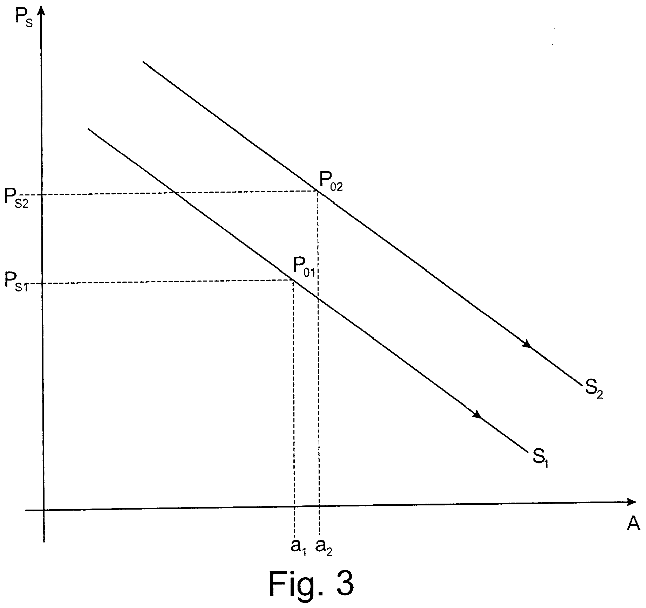

**Fig. 3** zeigt eine Diagrammdarstellung mit einem subjektiven, gemessenen intraokularen Druck auf der Ordinatenachse und einer Auslenkung einer Amplitude einer maximalen Verformung der Hornhaut 10 auf der Abzissenachse. Bei beispielsweise einem subjektiven intraokularen Druck auf Pₛ₁ und einer Amplitude a₁, welche dem Abstand X₂ entspricht, ergibt sich eine Steifigkeit S₁ als eine im Wesentlichen lineare Funktion mit negativer Steigung. Die Steifigkeit S₁ kann jedoch auch von einer linearen Funktion abweichen und als eine Linie mit einem vergleichsweise großen Krümmungsradius ausgebildet sein. Ein objektiver intraokularer Druck Pₒ₁ kann von der durch die Steifigkeit S₁ definierten Geraden als eine Variable entnommen werden. Analog dazu ergibt sich bei einem Druck Pₛ₂ und einer Auslenkung a₂ eine Parallelverschiebung der Geraden mit einer Steifigkeit S₂, woraus wieder ein objektiver intraokularer Druck Pₒ₂ abgeleitet werden kann. Alternativ können auch anstelle der Amplituden a₁ und a₂ die Durchmesser d₁ und d₂ in dem Diagramm eingesetzt und analog verwendet werden.

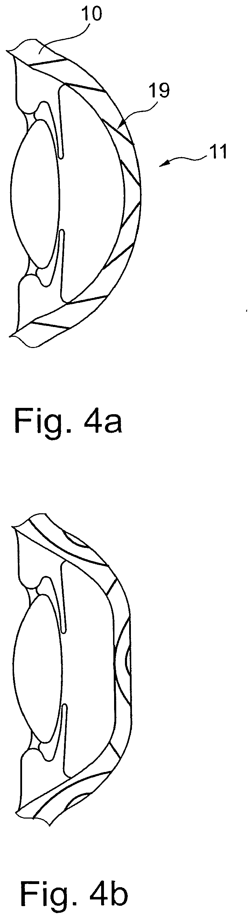

Die **Fig. 4a** bis **4b** zeigen die Verformungszustände der Hornhaut 10 des Auges 11 analog den **Fig. 1a** und **1b****.** Im Unterschied dazu sind bei den **Fig. 4a** und **4b** Spannungen im Material der Hornhaut visualisiert. So sind insbesondere Spannungslinien 19 im Material der Hornhaut 10 sichtbar, die Hauptspannungen längs und quer der Sehachse 12 repräsentieren. Die **Fig. 4a** zeigt somit Spannungen im Auge 11 in einem Ruhezustand der Hornhaut 10 und die **Fig. 4b** Spannungen im Auge 11 der verformten Hornhaut 10, die sich von den Spannungen im Ruhezustand wesentlich unterscheiden. Ein Vergleich der Spannung auf Basis der Spannungslinien 19 ermöglicht somit eine Definition einer Struktur- und/oder Materialeigenschaft der Hornhaut, die zur Korrektur eines gemessenen intraokularen Drucks und damit zur Ableitung eines objektiven intraokularen Drucks herangezogen werden kann.

## Patentansprüche

1. Ophthalmologisches Analyseverfahren zur Messung eines intraokularen Drucks in einem Auge (11) mit einem Analysesystem, gebildet aus einer Betätigungseinrichtung, mit der eine Hornhaut (10) des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei mit dem Beobachtungssystem Schnittbilder der unverformten und/oder verformten Hornhaut entlang einer Sehachse (12) des Auges aufgenommen werden, und einer Analyseeinrichtung, mit der aus den Schnittbildern der Hornhaut der intraokulare Druck abgeleitet wird, wobei in der Analyseeinrichtung aus den Schnittbildern der Hornhaut eine Struktureigenschaft der Hornhaut abgeleitet wird, wobei als Struktureigenschaft eine Spannung der Hornhaut abgeleitet wird, wobei Spannungen im Material der Hornhaut visualisiert werden, wobei als ein Schnittbild jeweils eine spannungsoptische Darstellung der Hornhaut verwendet wird, wobei aus Spannungslinien (19) der spannungsoptischen Darstellung die Struktureigenschaft der Hornhaut abgeleitet wird, wobei der intraokulare Druck unter Berücksichtigung der Spannungen der Hornhaut korrigiert wird.

2. Analyseverfahren nach Anspruch 1,
**dadurch gekennzeichnet,**
**dass** das Analysesystem in Art eines Polariskops ausgebildet ist, wobei das Beobachtungssystem eine Beleuchtungseinrichtung und eine Kameraeinrichtung umfasst, die jeweils einen Polarisator aufweisen, wobei mittels der Beleuchtungseinrichtung das Auge (11) mit linear, zirkular oder elliptisch polarisierten Licht beleuchtet wird.

3. Analyseverfahren nach Anspruch 2,
**dadurch gekennzeichnet,**
**dass** das Auge (11) mit monochromatischen oder polychromatischen Licht beleuchtet wird.

4. Analyseverfahren nach Anspruch 2 oder 3,
**dadurch gekennzeichnet,**
**dass** eine Polarisationsrichtung relativ zum Schnittbild gedreht wird.

5. Analyseverfahren nach einem der vorangehenden Ansprüche,
**dadurch gekennzeichnet,**
**dass** zur Ableitung der Struktureigenschaft eine Krümmung der unverformten und/oder verformten Hornhaut (10) aus den Schnittbildern der Hornhaut abgeleitet wird.

6. Analyseverfahren nach einem der vorangehenden Ansprüche,
**dadurch gekennzeichnet,**
**dass** zur Ableitung einer Materialeigenschaft bei Erreichen eines Applanationspunktes der Hornhaut (10) eine Größe einer ebenen Applanationsfläche gemessen wird, wobei ein Durchmesser der Applanationsfläche gemessen wird.

7. Analyseverfahren nach einem der vorangehenden Ansprüche,
**dadurch gekennzeichnet,**
**dass** eine vom intraokularen Druck unabhängige Materialeigenschaft der Hornhaut (10) abgeleitet wird, wobei der intraokulare Druck unter Berücksichtigung der Materialeigenschaften der Hornhaut abgeleitet wird.

8. Analyseverfahren nach Anspruch 7,
**dadurch gekennzeichnet,**
**dass** als eine Materialeigenschaft eine Steifigkeit der Hornhaut (10) abgeleitet wird, wobei der intraokulare Druck unter Berücksichtigung der Materialeigenschaften der Hornhaut abgeleitet wird.

9. Analyseverfahren nach Anspruch 7 oder 8,
**dadurch gekennzeichnet,**
**dass** eine Geschwindigkeit der bewegten Hornhaut (10) gemessen wird, wobei die Geschwindigkeit zur Ableitung einer weiteren Materialeigenschaft gemessen wird.

10. Analyseverfahren nach Anspruch 7 oder 8,
**dadurch gekennzeichnet,**
**dass** zur Ableitung der Materialeigenschaft ein Entfernungsmaß (X₂) einer maximalen Verformung der Hornhaut (10) relativ zum Apex (16) der Hornhaut aus den Schnittbildern der Hornhaut abgeleitet wird.

11. Analyseverfahren nach Anspruch 10,
**dadurch gekennzeichnet,**
**dass** eine Amplitude eines geometrischen Verformungsverlaufs der Hornhaut (10) aus den Schnittbildern der Hornhaut abgeleitet wird, wobei die Amplitude des Verformungsverlaufs zur Ableitung der Materialeigenschaft abgeleitet wird.

12. Analyseverfahren nach einem der Ansprüche 7 bis 11,
**dadurch gekennzeichnet,**
**dass** eine Lichtstreuung der Hornhaut (10) aus einem Schnittbild der Hornhaut abgeleitet wird, wobei aus der Lichtstreuung eines einzelnen Schnittbildes als eine weitere Materialeigenschaft eine Elastizität der Hornhaut abgeleitet wird.

13. Analyseverfahren nach einem der vorangehenden Ansprüche,
**dadurch gekennzeichnet,**
**dass** das Beobachtungssystem eine Kamera und eine Beleuchtungseinrichtung in einer Scheimpfluganordnung umfasst, wobei mittels der Kamera die Schnittbilder aufgenommen werden.

14. Ophthalmologisches Analysesystem zur Messung eines intraokularen Drucks in einem Auge (11), umfassend eine Betätigungseinrichtung, mit der eine Hornhaut (10) des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei mit dem Beobachtungssystem Schnittbilder der unverformten und/oder verformten Hornhaut entlang einer Sehachse (12) des Auges aufgenommen werden können, und eine Analyseeinrichtung, mit der aus den Schnittbildern der Hornhaut der intraokulare Druck abgeleitet werden kann, wobei die Analyseeinrichtung dazu eingerichtet ist, aus den Schnittbildern der Hornhaut eine Struktureigenschaft der Hornhaut abzuleiten, wobei als Struktureigenschaft eine Spannung der Hornhaut abgeleitet wird, wobei Spannungen im Material der Hornhaut visualisiert werden, wobei als ein Schnittbild jeweils eine spannungsoptische Darstellung der Hornhaut verwendet wird, wobei aus Spannungslinien (19) der spannungsoptischen Darstellung die Struktureigenschaft der Hornhaut abgeleitet wird, wobei der intraokulare Druck unter Berücksichtigung der Spannungen der Hornhaut korrigiert wird.

## Claims

1. An ophthalmological analysis method for measuring an intraocular pressure in an eye (11) using an analysis system consisting of an actuating device with which a cornea (10) of the eye is deformed in a contactless manner, wherein the actuating device causes an air puff to be applied to the eye in such a manner that the cornea is deformed, an observation system with which the deformation of the cornea is observed and recorded, wherein sectional images of the cornea when it is not deformed and/or deformed are created along a visual axis (12) of the eye with the observation system, and an analysis device with which the intraocular pressure is derived from the sectional images of the cornea, wherein a structural characteristic of the cornea is derived from the sectional images of the cornea in the analysis device,
wherein a corneal stress is derived as a structural characteristic, wherein stresses are visualised in the material of the cornea, wherein one photoelastic representation of the cornea is used as a sectional image in each case, wherein the structural characteristic of the cornea is derived from stress lines (19) of the photoelastic representation, wherein the intraocular pressure is corrected taking the corneal stresses into account.

2. The analysis method as recited in claim 1,
**characterised in that**
the analysis system is designed in the manner of a polariscope, wherein the observation system comprises an illumination device and a camera device, each of which is equipped with a polariser, wherein the eye (11) is illuminated with linearly, circularly or elliptically polarised light via the illumination device.

3. The analysis method as recited in claim 2,
**characterised in that**
the eye (11) is illuminated with monochromatic or polychromatic light.

4. The analysis method as recited in claim 2 or 3,
**characterised in that**
a polarisation direction is rotated relative to the sectional image.

5. The analysis method as recited in any of the preceding claims,
**characterised in that**
a curvature of the cornea (10) when it is not deformed and/or deformed is derived from the sectional images of the cornea for the purpose of deriving the structural characteristic.

6. The analysis method as recited in any of the preceding claims,
**characterised in that**
a size of a flat applanation area is measured when an applanation point of the cornea (10) is reached for the purpose of deriving a material characteristic, wherein a diameter of the applanation area is measured.

7. The analysis method as recited in any of the preceding claims,
**characterised in that**
a material characteristic of the cornea (10) that is independent of the intraocular pressure is derived, wherein the intraocular pressure is derived taking the material characteristics of the cornea into account.

8. The analysis method as recited in claim 7,
**characterised in that**
a rigidity of the cornea (10) is derived as a material characteristic, wherein the intraocular pressure is derived taking the material characteristics of the cornea into account.

9. The analysis method as recited in claim 7 or 8,
**characterised in that**
a speed of the movement of the cornea (10) is measured, wherein the speed is measured for the purpose of deriving another material characteristic.

10. The analysis method as recited in claim 7 or 8,
**characterised in that**
a distance (X₂) of a maximum deformation of the cornea (10) relative to the apex (16) of the cornea is derived from the sectional images of the cornea for the purpose of deriving the material characteristic.

11. The analysis method as recited in claim 10,
**characterised in that**
an amplitude of a geometrical deformation progression of the cornea (10) is derived from the sectional images of the cornea, wherein the amplitude of the deformation progression is derived for the purpose of deriving the material characteristic.

12. The analysis method as recited in any of the claims 7 to 11,
**characterised in that**
a scattering of light by the cornea (10) is derived from a sectional image of the cornea, wherein an elasticity of the cornea is derived from the light scattering of a single sectional image as another material characteristic.

13. The analysis method as recited in any of the preceding claims,
**characterised in that**
the observation system comprises a camera and an illumination device in a Scheimpflug arrangement, wherein the sectional images are taken with the camera.

14. An ophthalmological analysis system for measuring an intraocular pressure in an eye (11), comprising an actuating device with which a cornea (10) of the eye can be deformed in a contactless manner, wherein the actuating device causes an air puff to be applied to the eye in such a manner that the cornea is deformed, an observation system with which the deformation of the cornea can be observed and recorded, wherein sectional images of the cornea when it is not deformed and/or deformed can be created along a visual axis (12) of the eye with the observation system, and an analysis device with which the intraocular pressure can be derived from the sectional images of the cornea, wherein the analysis device is designed for deriving a structural characteristic of the cornea from the sectional images of the cornea,
wherein a corneal stress is derived as a structural characteristic, wherein stresses are visualised in the material of the cornea, wherein one photoelastic representation of the cornea is used as a sectional image in each case, wherein the structural characteristic of the cornea is derived from stress lines (19) of the photoelastic representation, wherein the intraocular pressure is corrected taking the corneal stresses into account.

## Revendications

1. Procédé d'analyse ophtalmologique destiné à mesurer une pression intraoculaire dans un œil (11) à l'aide d'un système d'analyse, consistant en une unité d'actionnement à l'aide de laquelle une cornée (10) de l'œil est déformée sans contact, ladite unité d'actionnement dirigeant un jet d'air sur l'œil pour déformer la cornée, en un système d'observation à l'aide duquel la déformation de la cornée est observée et acquise, ledit système d'observation acquérant des images en coupe de la cornée non déformée et/ou déformée le long d'un axe visuel (12) de l'œil, et en une unité d'analyse à l'aide de laquelle la pression intraoculaire est dérivée des images en coupe de la cornée, une propriété structurelle de la cornée étant dérivée des images en coupe de la cornée dans l'unité d'analyse, une tension de la cornée étant dérivée comme propriété structurelle, des tensions étant visualisées dans le matériau de la cornée, une représentation photoélastique de la cornée respectivement étant utilisée comme image en coupe chaque fois, ladite propriété structurelle de la cornée étant dérivée de lignes de tension (19) de la représentation photoélastique, la pression intraoculaire étant corrigée en tenant compte des tensions de la cornée.

2. Procédé d'analyse selon la revendication 1,
**caractérisé en ce que**
le système d'analyse est réalisé à la manière d'un polariscope, le système d'observation comprenant une unité d'illumination et une unité de caméra, chacune desdites unités présentant un polariseur, l'œil (11) étant illuminé avec de la lumière polarisée linéairement, circulairement ou elliptiquement moyennant l'unité d'illumination.

3. Procédé d'analyse selon la revendication 2,
**caractérisé en ce que**
l'œil (11) est illuminé avec de la lumière monochromatique ou polychromatique.

4. Procédé d'analyse selon la revendication 2 ou 3,
**caractérisé en ce**
**qu'**une direction de la polarisation est tournée par rapport à l'image en coupe.

5. Procédé d'analyse selon l'une quelconque des revendications précédentes,
**caractérisé en ce**
**qu'**une courbure de la cornée (10) non déformée et/ou déformée est dérivée des images en coupe de la cornée pour dériver la propriété structurelle.

6. Procédé d'analyse selon l'une quelconque des revendications précédentes,
**caractérisé en ce que**
pour dériver une propriété de matériau, une dimension d'une surface d'applanation plane est mesurée quand un point d'applanation de la cornée (10) est atteint, un diamètre de la surface d'applanation étant mesuré.

7. Procédé d'analyse selon l'une quelconque des revendications précédentes,
**caractérisé en ce**
**qu'**une propriété du matériau de la cornée (10) qui est indépendante de la pression intraoculaire est dérivée, la pression intraoculaire étant dérivée en tenant compte des propriétés du matériau de la cornée.

8. Procédé d'analyse selon la revendication 7,
**caractérisé en ce**
**qu'**une rigidité de la cornée (10) est dérivée comme propriété de matériau, la pression intraoculaire étant dérivée en tenant compte des propriétés du matériau de la cornée.

9. Procédé d'analyse selon la revendication 7 ou 8,
**caractérisé en ce**
**qu'**une vitesse de la cornée (10) mue est mesurée, ladite vitesse étant mesurée pour dériver une autre propriété de matériau.

10. Procédé d'analyse selon la revendication 7 ou 8,
**caractérisé en ce**
**qu'**une dimension de distance (X₂) d'une déformation maximale de la cornée (10) par rapport à l'apex (16) de la cornée est dérivée des images en coupe de la cornée pour dériver la propriété de matériau.

11. Procédé d'analyse selon la revendication 10,
**caractérisé en ce**
**qu'**une amplitude d'un déroulement géométrique de la déformation de la cornée (10) est dérivée des images en coupe de la cornée, ladite amplitude du déroulement de la déformation étant dérivée pour dériver la propriété de matériau.

12. Procédé d'analyse selon l'une quelconque des revendications 7 à 11,
**caractérisé en ce**
**qu'**une diffusion de lumière de la cornée (10) est dérivée d'une image en coupe de la cornée, une élasticité de la cornée étant dérivée de la diffusion de lumière d'une image en coupe individuelle comme autre propriété de matériau.

13. Procédé d'analyse selon l'une quelconque des revendications précédentes,
caractérisé en ce
le système d'observation comprend une caméra et une unité d'illumination dans une configuration de Scheimpflug, les images en coupe étant acquises moyennant la caméra.

14. Système d'analyse ophtalmologique destiné à mesurer une pression intraoculaire dans un œil (11), comprenant une unité d'actionnement à l'aide de laquelle une cornée (10) de l'œil peut être déformée sans contact, ladite unité d'actionnement pouvant diriger un jet d'air sur l'œil pour déformer la cornée, un système d'observation à l'aide duquel la déformation de la cornée peut être observée et acquise, ledit système d'observation pouvant acquérir des images en coupe de la cornée non déformée et/ou déformée le long d'un axe visuel (12) de l'œil, et une unité d'analyse à l'aide de laquelle la pression intraoculaire peut être dérivée des images en coupe de la cornée, ladite unité d'analyse étant configurée pour dériver une propriété structurelle de la cornée des images en coupe de la cornée, une tension de la cornée étant dérivée comme propriété structurelle, des tensions étant visualisées dans le matériau de la cornée, une représentation photoélastique de la cornée étant utilisée comme image en coupe chaque fois, ladite propriété structurelle de la cornée étant dérivée de lignes de tension (19) de la représentation photoélastique, la pression intraoculaire étant corrigée en tenant compte des tensions de la cornée.
